# EUROPEAN PATENT APPLICATION

(11) **EP 4 706 630 A2**
(43) Date of publication of application: **11.03.2026**
(21) Application number: 25221501.7
(22) Date of filing: 17.05.2022
(51) Int. Cl.: A61K 8/34

(54) **SUNSCREEN COMPOSITION COMPRISING BEMOTRIZINOL**

(30) Priority: 19.05.2021 US 202163190507 P; 08.06.2021 EP 21178148
(62) Divisional of application: 22729209.1
(71) Applicant: DSM IP Assets B.V., 6221 BE Maastricht (NL)
(72) Inventor: DWYER, Margaret, Kaiseraugst (CH); JANSSEN, Anne, Kaiseraugst (CH); KUNZE, Gernot Ulrich, Kaiseraugst (CH); UTTEMBERGUE D ELlA, Luciana, Kaiseraugst (CH); RADOMSKY, Karina, Kaiseraugst (CH); RUDOLPH, Thomas, Kaiseraugst (CH); VOLLHARDT, Juergen Herbert, Kaiseraugst (CH)
(74) Representative: dsm-firmenich IP

(57) **Abstract**

The present invention relates to sunscreen compositions containing bis-ethylhexyloxyphenol methoxyphenyl triazine (BEMT) and at least one film forming agent selected from the group consisting of citrate crosspolymers and polyamides in combination with an alkandiol with the proviso that the composition is free of octocrylene, ethylhexyl triazone, diethylamino hydroxybenzoyl hexyl benzoate and ethylhexyl methoxycinnamate. Said compositions exhibit a reduced soaping effect and thus a more appealing skin feel and look.

## Description

The present invention relates to sunscreen compositions containing bis-ethylhexyloxyphenol methoxyphenyl triazine (BEMT) and at least one film forming agent selected from the group consisting of citrate crosspolymers and polyamides in combination with an alkandiol with the proviso that the composition is free of octocrylene, ethylhexyl triazone, diethylamino hydroxybenzoyl hexyl benzoate and ethylhexyl methoxycinnamate. Said compositions exhibit a reduced soaping effect and thus a more appealing skin feel and look.

Sunscreens should be effective and safe. In this respect sunscreens contain different UVB and UVA filter. Among these UV-filters bis-ethylhexyloxyphenol methoxyphenyl triazine (BEMT) is particularly advantageous as it is a broad-spectrum UV absorber, absorbing UVB as well as UVA rays, is highly photostable, has strong synergistic effects on the SPF e.g. when formulated with bisoctrizole or iscotrizinol. Overall it is currently regarded the most effective UV absorber available measured by SPF, based on the maximum concentration permitted by European legislation.

However sunscreens comprising bis-ethylhexyloxyphenol methoxyphenyl triazine tend to exhibit a soaping effect. To prevent this, consumers tend to use less sunscreen but at the same time they are less protected. The adverse effects of UV radiation on skin are well known. It is therefore important for the industry to provide solutions for such kind of issues to guarantee, that consumers apply sufficient sunscreen for appropriate protection.

It was therefore the object of the present invention to remedy the disadvantages of the prior art and to develop sun care products comprising bis-ethylhexyloxyphenol methoxyphenyl triazine (BEMT) which exhibit a reduced soaping effect thus resulting in a more appealing skin feel.

Furthermore, such sunscreens should be suitable for the US market and not contain UV-filter substances which despite their approval by the approval authorities, are not without controversy which may lead to a downgrading by some consumer magazines (e.g. eco-test).

Surprisingly, it has now be found, that the use of certain film formers is able to reduce said soaping effect.

Thus, in a first embodiment, the present invention provides sunscreen compositions comprising bis-ethylhexyloxyphenol methoxyphenyl triazine (BEMT), at least one film forming agent selected from the group consisting of citrate polyesters and polyamides with the proviso that the compositions are free of octocrylene, ethylhexyl triazone, diethylamino hydroxybenzoyl hexyl benzoate and ethylhexyl methoxycinnamate. In a preferred embodiment said composition further comprises at least one alkanediol.

Another subject matter of the invention is directed to a method of reducing the soaping effect of sunscreen compositions comprising bis-ethylhexyloxyphenol methoxyphenyl triazine, said method comprising the step of adding at least one film forming agent selected from the group consisting of citrate polyesters and polyamides into said composition and optionally appreciating the effect.

In a further embodiment the present invention also relates to the use of at least one film forming agent selected from the group consisting of citrate polyesters and polyamides to reduce the soaping effect of bis-ethylhexyloxyphenol methoxyphenyl triazine in sunscreen compositions.

Bis-Ethylhexyloxyphenol methoxyphenyl triazine (also referred to herein as BEMT) is also known as 2,4-bis{[4-(2-ethylhexyloxy)-2-hydroxy]phenyl}-6-(4-methoxyphenyl)-1,3,5-triazine (chemical name) or bemotrizinol (INN). BEMT acts as a broad-spectrum UV filter absorbing UVB as well as UVA rays. It has two absorption peaks, 310 and 340 nm. BEMT is suggested for use in sun, day care, alphabetic products such as BB cream and whitening products.

In all embodiments of the present invention, the amount of BEMT in the sunscreen compositions according to the present invention is advantageously selected in the range from 0.4 to 10 wt.-%., preferably in the range from 0.4 to 9 wt.-%, 0.4 to 8 wt.-%, 0.4 to 7 wt.-%, 0.4 to 6 wt.-%, 0.4 to 5 wt.-%, 0.4 to 4 wt.-%, 0.4 to 3 wt.-%, 0.5 to 3 wt.-%, 0.8 to 9 wt.-%, 0.8 to 8 wt.-%, 0.8 to 7 wt.-%, 0.8 to 6 wt.-%, 0.8 to 5 wt.-%, 0.8 to 4 wt.-%, 0.8 to 3 wt.-%, such as for instance in the range from 1 to 5 wt.-%, from 1 to 3 wt.-%, from 2 to 5 wt.-% or from 2 to 4 wt.-%, based on the total weight of the composition.

Citrate polyesters according to the present invention are characterized by including citric acid, alcohols of different chain lengths (stearyl C₁₈, octyldodecyl C₂₀ and behenyl C₂₂) and a crosslinking reagent. Preferred citrate ester polymers according to the present invention are (INCI names) octyldodecyl citrate crosspolymer, stearyl/octyldodecyl citrate crosspolymer and behenyl/octyldodecyl propanediol citrate crosspolymer. Said polymers are commercially available under the tradename CosmoSurf^{®} at Ultra Chemical Inc., USA. Most preferably, the citrate polyester according to the present invention is stearyl/ octyldodecyl citrate crosspolymer, commercially available CosmoSurf^{®} CE-140.

Preferred polyamides according to the present invention encompass (INCI names) polyamide-3, polyamide-4, as well as polyamide-8. Said polymers are e.g. commercially available under the tradename Oleocraft^{™} from Croda. Most preferably, the polyamide according to the present invention is polyamide-8 commercially available as Oleocraft L-20 from Croda.

Preferred film forming agents in all embodiments of the present invention are the polyamides as they provide a more pronounced reduction in the soaping effect. Most preferred in all embodiments of the present invention is the use of polyamide-8 as film forming agent, even more preferably as sole film forming agent.

In all embodiments of the present invention, the (total) amount of the film forming agent in the sunscreen compositions according to the present invention is advantageously at least 0.1 wt.% and at most 10 wt.-%. Preferably the amount is selected in the range from 0.5 to 10 wt.-%, more preferably in the range from 0.75 to 6 wt.-%, most preferably from 1 to 5 wt.-%, such as from 1 to 4 wt.-%, from 2 to 4 wt.-% or from 2.5 to 3.5 wt.-%, based on the total weight of the composition.

In all embodiments of the present invention, the ratio of the total amount of the at least one film forming polymer to the total amount of BEMT is preferably selected in the range from 0.1:5 to 5:0.1, preferably in the range from 0.5:2.5 to 2.5:0.5, most preferably in the range from 0.5:1 to 1:0.5.

In a preferred embodiment, the sunscreen compositions of the present invention further comprise one or more alkanediol. Suitable alkanediols encompass 1,2-alkanediols and 1,3-alkandiols such as in particular 1,2-butandiol, butylene glycol (1,3-butandiol), 1,2-pentanediol, 1,2-hexanediol, 2-methyl-1,3-propanediol and 1,3-propanediol. Preferred in all embodiments of the present invention is the use of 1,3-propanediol, butyleneglycol and/ or 1,2-butandiol. Most preferred in all embodiments of the present the composition comprises as alkanediol 1,3-propandiol, butylene glycol and/ or 1,2-butandiol, preferably in the absence of any further alkanediols. Even more preferably, the compositions do not comprise any 1,3-propanediol as this further reduces the soaping effect. Even more preferably, only butylene glycol is contained in the formulation as sole alkanediol.

Thus, in a particular advantageous embodiment, the compositions comprise as sole alkanediol(s) either
a) 1,3-propanediol and butylene glycol,
b) 1,3-propandiol, or
c) butylene glycol.

The amount of the alkanediol(s) in the sunscreen compositions according to the present invention is preferably selected in the range from 1 to 10 wt.-%, more preferably from 1.5 to 7.5 wt.-%, most preferably from 2 to 6 wt.-%, based on the total weight of the composition.

As the sunscreen compositions according to the invention are intended for topical application, it is well understood that they comprise a physiologically acceptable medium, i.e. a medium compatible with keratinous substances, such as the skin, mucous membranes, and keratinous fibres. In particular, the physiologically acceptable medium is a cosmetically acceptable carrier.

It is well understood, that the term sunscreen composition as used herein refers to composition comprising at least BEMT, preferably in the presence of further substances which absorb UV-light such as commonly used UVA, UVB or broadband UV-filter substances.

In all embodiments of the present invention it is preferred that the compositions are free of octocrylene, ethylhexyl triazone, diethylamino hydroxybenzoyl hexyl benzoate and ethylhexyl methoxycinnamate.

The term 'cosmetically acceptable carrier' as used herein refers to all carriers and/or excipients and/ or diluents conventionally used in topical cosmetic compositions such as in particular in skin care preparations.

The exact amount of carrier will depend upon the actual level of the UV-filter substances and any other optional ingredients that one of ordinary skill in the art would classify as distinct from the carrier (e.g., other active ingredients).

In an advantageous embodiment, the sunscreen compositions according to the present invention comprise from 50% to 99%, preferably from 60% to 98%, more preferably from 70% to 98%, such as in particular from 80% to 95% of a carrier, based on the total weight of the sunscreen composition.

In a particular advantageous embodiment, the carrier consists furthermore of at least 30 wt.-%, more preferably of at least 40 wt.-%, most preferably of at least 45 wt.-% of water, such as in particular of 50 to 90 wt.-% of water. Further suitable ranges are from 50 to 75 wt.-%, from 50 to 70 wt.-%, and from 55 to 60 wt.-%.

In particular, the sunscreen compositions according to the present invention are cosmetic or pharmaceutical compositions, preferably cosmetic (non-therapeutic) compositions.

In one embodiment, the sunscreen compositions according to the present invention are applied to mammalian keratinous tissue such as in particular to human skin or the human scalp and hair.

The term "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic compositions as disclosed in A. Domsch, "Cosmetic Compositions", Verlag für chemische Industrie (ed. H. Ziolkowsky), 4th edition, 1992.

Preferred sunscreen compositions according to the invention are skin care preparations, decorative preparations, and functional preparations.

Examples of skin care preparations are, in particular, light protective preparations, anti-ageing preparations, preparations for the treatment of photo-ageing, body oils, body lotions, body gels, treatment creams, skin protection ointments, skin powders, sticks, balms, moisturizing gels, moisturizing sprays, face and/or body moisturizers, skin-tanning preparations (i.e. compositions for the artificial/sunless tanning and/or browning of human skin), for example self-tanning creams as well as skin lightening preparations.

Examples of decorative preparations are, in particular, lipsticks, eye shadows, mascaras, dry and moist make-up formulations, rouges and/or powders.

Examples of functional preparations are cosmetic or pharmaceutical compositions containing active ingredients such as hormone preparations, vitamin preparations, vegetable extract preparations, anti-ageing preparations, and/or antimicrobial (antibacterial or antifungal) preparations without being limited thereto.

In a particular embodiment, the sunscreen compositions according to the invention are light-protective preparations (sun care products, sunscreens), such as sun protection milks, sun protection lotions, sun protection creams, sun protection oils, sun blocks or topical's or day care creams with or without a SPF (sun protection factor) label. Of particular interest are sun protection creams, sun protection lotions and sun protection milks.

The compositions of the invention (including the carrier) may comprise conventional adjuvants and additives, such as preservatives/antioxidants, fatty substances/oils, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

In accordance with the present invention, the compositions according to the invention may comprise further ingredients such as ingredients for skin lightening; tanning prevention; treatment of hyperpigmentation; preventing or reducing acne, wrinkles, lines, atrophy and/or inflammation; chelators and/or sequestrants; anti-cellulites and slimming (e.g. phytanic acid), firming, moisturizing and energizing, self-tanning, soothing agents, as well as agents to improve elasticity and skin barrier and carriers and/or excipients or diluents conventionally used in sunscreen compositions.

If nothing else is stated, the excipients, additives, diluents, etc. mentioned in the following are suitable for sunscreen compositions according to the present invention. The necessary amounts of the cosmetic and dermatological adjuvants and additives can, based on the desired product, easily be determined by the skilled person.

The additional ingredients can either be added to the oily phase, the aqueous phase or separately as deemed appropriate. The mode of addition can easily be adapted by a person skilled in the art.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

Preferred sunscreen compositions in all embodiments of the present invention are emulsions containing an oily phase and an aqueous phase such as in particular an O/W, W/O, Si/W, W/Si, O/W/O, W/O/W multiple or a pickering emulsions. The amount of the oily phase (i.e. the phase containing all oils and fats) present in such emulsions is preferably at least 10 wt.--%, such as in the range from 10 to 60 wt.-%, preferably in the range from 15 to 50 wt.-%, most preferably in the range from 15 to 40 wt.-%, based on the total weight of the composition.

According to one even more preferred embodiment, the sunscreen compositions according to the present invention as outlined herein are O/W emulsions comprising an oily phase dispersed in an aqueous phase in the presence of an O/W emulsifier. The preparation of such O/W emulsions is well known to a person skilled in the art and illustrated in the examples.

In one advantageous embodiment, the O/W emulsifier is a phosphate ester emulsifier. Among the preferred phosphate ester emulsifier are C8-10 Alkyl Ethyl Phosphate, C9-15 Alkyl Phosphate, Ceteareth-2 Phosphate, Ceteareth-5 Phosphate, Ceteth-8 Phosphate, Ceteth-10 Phosphate, Cetyl Phosphate, C6-10 Pareth-4 Phosphate, C12-15 Pareth-2 Phosphate, C12-15 Pareth-3 Phosphate, DEA-Ceteareth-2 Phosphate, DEA-Cetyl Phosphate, DEA-Oleth-3 Phosphate, Potassium cetyl phosphate, Deceth-4 Phosphate, Deceth-6 Phosphate and Trilaureth-4 Phosphate. A particular phosphate ester emulsifier according to the invention is potassium cetyl phosphate e.g. commercially available as Amphisol^{®} K at DSM Nutritional Products Ltd Kaiseraugst.

Further suitable O/W emulsifiers according to the present invention encompass PEG 30 Dipolyhydroxystearate, PEG-4 Dilaurate, PEG-8 Dioleate, PEG-40 Sorbitan Peroleate, PEG-7 Glyceryl Cocoate, PEG-20 Almond Glycerides, PEG-25 Hydrogenated Castor Oil, Glyceryl Stearate (and) PEG-100 Stearate , PEG-7 Olivate, PEG-8 Oleate, PEG-8 Laurate, PEG-60 Almond Glycerides, PEG-20 Methyl Glucose Sesquistearate, PEG-40 Stearate, PEG-100 Stearate, PEG-80 Sorbitan Laurate, Steareth-2, Steareth-12, Oleth-2, Ceteth-2, Laureth-4, Oleth-10, Oleth-10/Polyoxyl 10 Oleyl Ether, Ceteth-10, Isosteareth-20, Ceteareth-20, Oleth-20, Steareth-20, Steareth-21, Ceteth-20, Isoceteth-20, Laureth-23, Steareth-100, glycerylstearatcitrate, glycerylstearate (self-emulsifying), stearic acid, salts of stearic acid, polyglyceryl-3-methylglycosedistearate. Further suitable emulsifiers are sorbitan oleate, sorbitan sesquioleate, sorbitan isostearate, sorbitan trioleate, Lauryl Glucoside, Decyl Glucoside, Sodium Stearoyl Glutamate, Sucrose Polystearate and Hydrated Polyisobuten. Furthermore, one or more synthetic polymers may be used as an emulsifier. For example, PVP eicosene copolymer, acrylates/C10-30 alkyl acrylate crosspolymer, acrylates/steareth-20 methacrylate copolymer, PEG-22/dodecyl glycol copolymer, PEG-45/dodecyl glycol copolymer, and mixtures thereof.

Another particular suitable class of O/W emulsifiers are non-ionic self-emulsifying system derived from olive oil e.g. known as (INCI Name) cetearyl olivate and sorbitan olivate (Chemical Composition: sorbitan ester and cetearyl ester of olive oil fatty acids) sold under the tradename OLIVEM 1000.

Further suitable are commercially available polymeric emulsifiers such as hydrophobically modified polyacrylic acid such as Acrylates/C10-30 Alkyl Acrylate Crosspolymers which are commercially available under the tradename Pemulen^{®} TR-1 and TR-2 by Noveon.

Another class of particularly suitable emulsifiers are polyglycerol esters or diesters of fatty acids also called polyglyceryl ester/ diester (i.e. a polymer in which fatty acid(s) is/ are bound by esterification with polyglycerine), such as e.g. commercially available at Evonik as Isolan GPS [INCI Name Polyglyceryl-4 Diisostearate/Polyhydroxystearate/Sebacate (i.e. diester of a mixture of isostearic, polyhydroxystearic and sebacic acids with Polyglycerin-4)] or Dehymuls PGPH available at Cognis (INCI Polyglyceryl-2 Dipolyhydroxystearate).

Also suitable are polyalkylenglycolether such as Brij 72 (Polyoxyethylen(2)stearylether) or Brij 721 (Polyoxyethylene (21) Stearyl Ether e.g. available at Croda.

Particularly advantageous O/W emulsifiers according to the present invention are one or more of Polyglyceryl-3 Methylglucose Distearate, Lauryl Glucoside (and) Polyglyceryl-2 Dipolyhydroxystearate, Glyceryl Sterate Citrate, Sodium Cetearyl Sulfate, Cetearyl Glucoside; Polyglyceryl-6 Stearate (and) Polyglyceryl-6 Behenate, Cetearyl Olivate (and) Sorbitan Olivate, Arachidyl Alcohol (and) Behenyl Alcohol (and) Arachidyl Glucosides, Cetearyl Alcohol (and) Coco-Glucoside, Coco-Glucoside (and) Coconut Alcohol, PEG-100 Stearate (and) Glyceryl Stearate, Sodium Stearoyl Glutamate, Steareth-20, Steareth-21, Steareth-25, Steareth-2, Ceteareth-25 and Ceteareth-6 (all listed by their INCI names).

It is particularly preferred in accordance with the invention if the composition comprises potassium cetyl phosphate as emulsifier.

The at least one O/W respectively Si/W emulsifier is preferably used in an amount of 0.5 to 10 wt. % such as in particular in the range of 0.5 to 5 wt.-% such as most in particular in the range of 0.5 to 4 wt.-%, based on the total weight of the composition.

Suitable W/O- or W/Si-emulsifiers according to the present invention are polyglyceryl-2-dipolyhydroxystearat, PEG-30 dipolyhydroxystearat, cetyl dimethicone copolyol, polyglyceryl-3 diisostearate polyglycerol esters of oleic/isostearic acid, polyglyceryl-6 hexaricinolate, polyglyceryl-4-oleate, magnesium stearate, sodium stearate, potassium laurate, potassium ricinoleate, sodium cocoate, sodium tallowate, potassium castorate, sodium oleate, and mixtures thereof. Further suitable W/Si-emulsifiers are Lauryl Polyglyceryl-3 Polydimethylsiloxyethyl Dimethicone and/or PEG-9 Polydimethylsiloxyethyl Dimethicone and/or Cetyl PEG/PPG-10/1 Dimethicone and/or PEG-12 Dimethicone Crosspolymer and/or PEG/PPG-18/18 Dimethicone. The at least one W/O emulsifier is preferably used in an amount of about 0.001 to 10 wt.-%, more preferably in an amount of 0.2 to 7 wt.-% with respect to the total weigh of the composition.

The sunscreen compositions according to the present invention furthermore advantageously contain at least one co-surfactant such as e.g. selected from the group of mono- and diglycerides and/ or fatty alcohols. The co-surfactant is generally used in an amount selected in the range of 0.1 to 10 wt.-%, such as in particular in the range of 0.5 to 7 wt.-%, such as most in particular in the range of 1 to 5 wt.-%, based on the total weight of the composition. Particular suitable co-surfactants are selected from the list of alkyl alcohols such as cetyl alcohol (Lorol C16, Lanette 16), cetearyl alcohol (Lanette O), stearyl alcohol (Lanette 18), behenyl alcohol (Lanette 22), polyhydroxystearic acid, glyceryl stearate, glyceryl myristate (Estol 3650), hydrogenated coco-glycerides (Lipocire Na10) as well as mixtures thereof.

In all embodiments of the present invention, it is particular advantageous if the composition also comprises cetyl alcohol, stearyl alcohol and/or glycerylstearate, preferably stearyl alcohol.

Advantageous embodiments of the composition of the present invention also include those wherein the composition comprises one or more oils selected from dicaprylate/dicaprate, phenethyl benzoate, C₁₂-C₁₅ alkyl benzoate, dibutyl adipate, diisopropyl sebacates, dicaprylyl carbonate, di-C₁₂-₁₃ alkyl tartrates, diethylhexyl syringylidene malonates, hydrogenated castor oil dimerates, triheptanoin, C₁₂-₁₃ alkyl lactates, C₁₆₋₁₇ alkyl benzoates, propylheptyl caprylates, caprylic/capric triglycerides, diethylhexyl 2,6-naphthalates, octyldodecanol, ethylhexyl cocoates. Preferably, the composition according to the present invention comprises as oil(s) dibutyl adipate, phenethyl benzoate, dicaprylyl carbonate, C₁₂-C₁₅ alkylbenzoate, caprylyl carbonate, capric/caprylic triglyceride as well as mixtures thereof, most preferably dicaprylyl carbonate, dibutyladipate and C₁₂-C₁₅ alkylbenzoate.

In a still further advantageous aspect of the invention, the sunscreen compositions of the present invention further comprise a preservative and/ or a preservative booster, preferably selected from the group consisting of ethanol, phenoxyethanol, ethylhexylglycerin, hexylglycerin, glyceryl caprylate as well as mixtures thereof, most preferably selected from the group of phenoxyethanol and ethylhexyl glycerine as well as mixtures thereof. When present, the preservative respectively the preservative booster is preferably used in an amount of 0.01 to 2 wt. %, more preferably in an amount of 0.05 to 1.5 wt.-%, most preferably in an amount of 0.1 to 1.0 wt.-%, based on the total weight of the composition.

It is further advantageous in accordance with the invention if the composition of the invention comprises ethanol, phenoxyethanol and/or ethylhexylglycerin.

In another advantageous aspect, the sunscreen compositions according to the present invention are free of any parabenes, benzethoniumchlorid, piroctone olamine, lauroylarginat, methylisothiazolinon, chlormethylisothiazolinon, bronopol, benzalkoniumchloride, formaldehyd releasing compounds, salicylic acid, triclosan, DMDM hydantoin, chlorphenesin and IPBC (lodopropinylbutyl carbamate).

In another advantageous aspect, the sunscreen compositions according to the present invention are furthermore free of oxybenzone and/ or methylbenzylidenechamphor.

The sunscreen compositions according to the invention may further contain one or more emollients which soothe and soften the skin. As an example, the emollient may be silicone (dimethicone, cyclomethicone), vegetable oils (grape seed, sesame seed, jojoba, etc.), butters (cocoa butter, shea butter), and petrolatum derivatives (petroleum jelly, mineral oil).

In another aspect, the sunscreen composition of the invention may comprise one or more fragrances selected from limonene, citral, linalool, alpha-isomethylionone, geraniol, citronellol, 2-isobutyl-4-hydroxy-4-methyltetrahydropyran, 2-tert-pentylcyclohexyl acetate, 3-methyl-5-phenyl-1-pentanol, 7-acetyl-1,1,3,4,4,6-hexamethyltetralin, adipic diester, cinnamal, amyl salicylate, alpha-amylcinnamaldehyde, alpha-methylionone, butylphenylmethylpropional, cinnamal, amylcinnamyl alcohol, anise alcohol, benzoin, benzyl alcohol, benzyl benzoate, benzyl cinnamate, benzyl salicylate, bergamot oil, bitter orange oil, butylphenylmethylpropional, cardamom oil, cedrol, cinnamal, cinnamyl alcohol, citronellyl methylcrotonate, citrus oil, coumarin, diethyl succinate, ethyllinalool, eugenol, Evernia furfuracea extract, Evernia prunastri extract, farnesol, guaiacwood oil, hexylcinnamal, hexyl salicylate, hydroxycitronellal, lavender oil, lemon oil, linalyl acetate, mandarin oil, menthyl PCA, methyl hexadecan, nutmeg oil, rosemary oil, sweet orange oil, terpineol, tonkabean oil, triethyl citrate, vanillin.

The composition of the invention may advantageously comprise moisturizers. Moisturizers are compounds or mixtures of compounds which give cosmetic compositions the quality, after application to or distribution on the skin surface, of reducing the loss of moisture of the stratum corneum (Experimental determination by e.g transepidermal water loss (TEWL)) and/or of positively influencing the hydration of the stratum corneum and/or keeping an actual hydration state.

Non-limiting examples of advantageous moisturizers for use in the present invention include glycerol, lactic acid and/or lactates, especially sodium lactate, biosaccharide gum-1, glycine soya, ethylhexyloxyglycerol, pyrrolidonecarboxylic acid, and urea. Of further advantage, in particular, is the use of polymeric moisturizers from the group of the polysaccharides which are water-soluble and/or swellable in water and/or gellable with the aid of water. Especially advantageous, for example, are hyaluronic acid, chitosan and/or a fucose-rich polysaccharide which is registered in Chemical Abstracts under the registry number 178463-23-5 and is available, for example, under the Fucogel^{®}1000 name from the company SOLABIA S.A. Moisturizers may also be used advantageously as active antiwrinkle ingredients for protection from changes to the skin of the kind occurring in skin aging, for example.

The cosmetic compositions of the invention may further comprise advantageously, although not mandatorily, fillers which have the effect, for example, of further improving the sensorial and cosmetic properties of the formulations and evoking or intensifying a velvety or silky skin sensation, for example. Advantageous fillers in the sense of the present invention are starch and starch derivatives (such as tapioca starch, distarch phosphate, aluminum or sodium starch octenylsuccinate, and the like, for example), Valvance pigments which have neither primarily UV filter effect nor coloring effect (such as Valvance Touch 210 or 250 for example) and/or Aerosils^{®} and/or talc and/or polyethylene, nylon, and silica dimethyl silylate.

The water phase of the compositions of the invention may advantageously comprise customary cosmetic auxiliaries, such as, for example, alcohols, particularly those of low C number, preferably ethanol and/or isopropanol, or polyols of low C number, and also ethers thereof, preferably glycerol, electrolytes, self-tanning agents, and also, in particular, one or more thickeners, which may be advantageously selected from the group of silicon dioxide, aluminum silicates, polysaccharides and/or derivatives thereof, e.g., hyaluronic acid, xanthan gum, hydroxypropylmethylcellulose, particularly advantageously from the group of polyacrylates, preferably a polyacrylate from the group referred to as carbopols, examples being carbopols of types 980, 981, 1382, 2984, and 5984, in each case individually or in combination. Further thickeners advantageous in accordance with the invention are those having the INCI designation Acrylates/C10-30 Alkyl Acrylate Crosspolymer (e.g., Pemulen TR 1, Pemulen TR 2, Carbopol 1328 from NOVEON) and also Aristoflex AVC (INCI: Ammonium Acryloyldimethyltaurate/VP Copolymer) as well as Simugel NS (INCI: Hydroxyethyl Acrylate/Sodium Acryloyldimethyl Taurate Copolymer & Squalane & Polysorbate 60).

It is preferred in accordance with the invention if the composition comprises xanthan gum, crosslinked acrylate/C10-C30 alkyl acrylate polymer, hydroxyethyl acrylate/sodium acryloyldimethyl taurate copolymer and/or vinylpyrrolidone/hexadecane copolymer, preferably xanthan gum and/ or hydroxyethyl acrylate/sodium acryloyldimethyl taurate, most preferably both of xanthan gum and hydroxyethyl acrylate/sodium acryloyldimethyl taurate.

The sunscreen compositions of the invention manage with a surprisingly small total amount of UV filters.

In another aspect, the composition may have an SPF of at least 15, preferably at least 20, most preferably of at least 30.

Advantageously in accordance with the invention, the composition of the invention may comprise further film formers.

It is especially advantageous to select said further film formers from the group of the polymers based on polyvinylpyrrolidone (PVP).

Particular preference is given to copolymers of vinylpyrrolidone, as for example the PVP hexadecane copolymer and the PVP eicosene copolymer, which are available under the trade names Antaron V216 and Antaron V220 from GAF Chemicals Corporation.

Likewise advantageous as further polymeric film formers are for example, sodium polystyrene sulfonate, which is available under the trade name Flexan 130 from National Starch and Chemical Corp., and/or polyisobutene, available from Rewo under the trade name Rewopal PIB1000. Examples of further suitable polymers are polyacrylamides (Seppigel 305), polyvinyl alcohols, PVP, PVPNA copolymers, polyglycols, acrylate/octylacrylamide copolymer (Dermacryl 79) Likewise advantageous is the use of hydrogenated castor oil dimer dilinoleate (INCI Hydrogenated Castor Oil Dimer Dilinoleate), which can be acquired from Kokyu Alcohol Kogyo under the name Risocast DA-H, or else PPG-3 benzyl ether myristate, which can be acquired under trade name Crodamol STS from Croda Chemicals.

The sunscreen compositions according to the invention in general have a pH in the range of 3 to 10, preferably a pH in the range of 4 to 8 and most preferably a pH in the range of 4 to 7. The pH can easily be adjusted as desired with suitable acids such as e.g. citric acid or bases such as NaOH according to standard methods in the art.

In accordance with the invention is the use of the composition of the invention for protection from skin aging (especially for protection from UV-induced skin aging) and also as a sun protection composition.

Finally, a subject-matter of the invention is a method for the cosmetic treatment of keratinous substances such as in particular the skin, wherein a composition as defined herein is applied to the said keratinous substances such as in particular to the skin. The method is in particular suitable to protect the skin against the adverse effects of UV-radiation such as in particular sun-burn and/ or photoageing while avoiding florescence stains on garment and/ or clothing.

The following examples are provided to further illustrate the compositions and effects of the present invention. These examples are illustrative only and are not intended to limit the scope of the invention in any way.

### Experimental Part

### Formulations

The formulations (O/W emulsions) as outlined in table 1 and 2 have been prepared according to standard methods in the art.

### Method

The soaping effect has been determined on LENETA black cards according to the method as outlined below:
- Apply 300mg of cream on LENETA black card
- cream is spread by a spreading device (speed: 10mm/s) using a 90mm spreading knife for homogenous film thickness
- LAB is measured by Minolta Chroma Meter CR-300 immediately after application, 4 measuring spots on each card
- average L value is calculated and compared

The results are outlined in Tables 1 to 4

**Table 1: Formulation I**

| **INCI** | **Ref 1** | **Inv 1** | **Inv 2** |
|---|---|---|---|
| DISODIUM EDTA; | 0.10 | 0.10 | 0.10 |
| XANTHAN GUM; | 0.25 | 0.25 | 0.25 |
| AQUA; | ad 100 | ad 100 | ad 100 |
| BUTYLENE GLYCOL; | 3.00 | 3.00 | 3.00 |
| STEARYL/OCTYLDODECYL CITRATE CROSSPOLYMER; | - | 3.00 | - |
| BIS-ETHYLHEXYLOXYPHENOL METHOXYPHENYL TRIAZINE; | 3.00 | 3.00 | 3.00 |
| POLYAMIDE-8; | - | - | 3.00 |
| STEARYL ALCOHOL; | 2.50 | 2.50 | 2.50 |
| POTASSIUM CETYL PHOSPHATE; | 2.50 | 2.50 | 2.50 |
| DIBUTYL ADIPATE; | 3.00 | 3.00 | 3.00 |
| DICAPRYLYL CARBONATE; | 13.00 | 13.00 | 13.00 |
| C12-15 ALKYL BENZOATE; | 15.00 | 15.00 | 15.00 |
| HYDROXYETHYL ACRYLATE/SODIUM ACRYLOYLDIMETHYL TAURATE COPOLYMER; | 0.50 | 0.50 | 0.50 |
| PHENOXYETHANOL; ETHYLHEXYLGLYCERIN; | 1.00 | 1.00 | 1.00 |
| **L value (average 4 spots)** | **31.2** | **26.1** | **25.2** |
| **Δ versus Ref 1** | **-** | **-16%** | **-19%** |

**Table 2: Formulation II**

| **INCI** | **Ref 2** | **Inv 3** | **Inv 4** |
|---|---|---|---|
| DISODIUM EDTA; | 0.10 | 0.10 | 0.10 |
| PROPANEDIOL; | 3.00 | 3.00 | 3.00 |
| XANTHAN GUM; | 0.25 | 0.25 | 0.25 |
| AQUA; | ad 100 | ad 100 | ad 100 |
| BUTYLENE GLYCOL; | 3.00 | 3.00 | 3.00 |
| STEARYL/OCTYLDODECYL CITRATE CROSSPOLYMER; | - | 3.00 | - |
| POLYAMIDE-8; | - | - | 3.00 |
| BIS-ETHYLHEXYLOXYPHENOL METHOXYPHENYL TRIAZINE; | 3.00 | 3.00 | 3.00 |
| STEARYL ALCOHOL; | 2.50 | 2.50 | 2.50 |
| POTASSIUM CETYL PHOSPHATE; | 2.50 | 2.50 | 2.50 |
| DIBUTYL ADIPATE; | 3.00 | 3.00 | 3.00 |
| DICAPRYLYL CARBONATE; | 13.00 | 13.00 | 13.00 |
| C12-15 ALKYL BENZOATE; | 15.00 | 15.00 | 15.00 |
| HYDROXYETHYL ACRYLATE/SODIUM ACRYLOYLDIMETHYL TAURATE COPOLYMER; | 0.50 | 0.50 | 0.50 |
| PHENOXYETHANOL; ETHYLHEXYLGLYCERIN; | 1.00 | 1.00 | 1.00 |
| **L value (average 4 spots)** | **31.2** | **28.8** | **26.7** |
| **A versus Ref** | **-** | **-7%** | **-14%** |

As can be retrieved from table 1 and 2, the compositions according to the present invention exhibit a significant reduction of the L value illustrating a reduced soaping effect, which effect is particularly pronounced when using polyamide-8 and/ or in the absence of 1,3-propanediol.

**Table 3: Formulation III**

| INCI | Inv 5 | Ref 3 | Ref 4 | Ref 5 |
|---|---|---|---|---|
| DISODIUM EDTA; | 0.10 | 0.10 | 0.10 | 0.10 |
| PROPANEDIOL; | 3.00 | 3.00 | | |
| XANTHAN GUM; | 0.25 | 0.25 | 0.25 | 0.25 |
| AQUA; | Ad 100 | Ad 100 | Ad 100 | Ad 100 |
| BIS-ETHYLHEXYLOXYPHENOL METHOXYPHENYL TRIAZINE; | 3.00 | 3.00 | 3.00 | 3.00 |
| POLYAMIDE-8; | 3.00 | 3.00 | 3.00 | 3.00 |
| Octocrylene | | 3.00 | | 3.00 |
| STEARYL ALCOHOL; | 2.50 | 2.50 | 2.50 | 2.50 |
| POTASSIUM CETYL PHOSPHATE; | 2.50 | 2.50 | 2.50 | 2.50 |
| DIBUTYL ADIPATE; | 3.00 | 3.00 | 3.00 | 3.00 |
| DICAPRYLYL CARBONATE; | 13.00 | 13.00 | 13.00 | 13.00 |
| C12-15 ALKYL BENZOATE; | 15.00 | 15.00 | 15.00 | 15.00 |
| HYDROXYETHYL ACRYLATE/SODIUM ACRYLOYLDIMETHYL TAURATE COPOLYMER; | 0.50 | 0.50 | 0.50 | 0.50 |
| PHENOXYETHANOL; ETHYLHEXYLGLYCERIN; | 1.00 | 1.00 | 1.00 | 1.00 |
| | | | | |
| L value (average 4 spots) | 26.9 | 27.5 | 30.9 | 31.9 |
| Δ versus Inv 5 | | + 2.2% | +15% | +19% |

**Table 4: Formulation IV**

| INCI | Inv 6 | Ref 6 | Ref 7 | Ref 8 |
|---|---|---|---|---|
| DISODIUM EDTA; | 0.10 | 0.10 | 0.10 | 0.10 |
| XANTHAN GUM; | 0.25 | 0.25 | 0.25 | 0.25 |
| AQUA; | Ad 100 | Ad 100 | Ad 100 | Ad 100 |
| BUTYLENE GLYCOL; | 3.00 | 3.00 | | |
| BIS-ETHYLHEXYLOXYPHENOL METHOXYPHENYL TRIAZINE; | 3.00 | 3.00 | 3.00 | 3.00 |
| POLYAMIDE-8; | 3.00 | 3.00 | 3.00 | 3.00 |
| Octocrylene | | 3.00 | | 3.00 |
| STEARYL ALCOHOL; | 2.50 | 2.50 | 2.50 | 2.50 |
| POTASSIUM CETYL PHOSPHATE; | 2.50 | 2.50 | 2.50 | 2.50 |
| DIBUTYL ADIPATE; | 3.00 | 3.00 | 3.00 | 3.00 |
| DICAPRYLYL CARBONATE; | 13.00 | 13.00 | 13.00 | 13.00 |
| C12-15 ALKYL BENZOATE; | 15.00 | 15.00 | 15.00 | 15.00 |
| HYDROXYETHYL ACRYLATE/SODIUM ACRYLOYLDIMETHYL TAURATE COPOLYMER; | 0.50 | 0.50 | 0.50 | 0.50 |
| PHENOXYETHANOL; ETHYLHEXYLGLYCERIN; | 1.00 | 1.00 | 1.00 | 1.00 |
| | | | | |
| L value (average 4 spots) | 25.6 | 27.0 | 30.9 | 31.9 |
| Δ versus Inv 6 | | + 5% | +20% | +24% |

As can be retrieved from table 3 and 4, the compositions according to the present invention exhibit a significant reduction of the L value illustrating a reduced soaping effect in the absence of octocrylene respectively in combination with the alkanediol.

## Claims

1. A sunscreen composition comprising bis-ethylhexyloxyphenol methoxyphenyl triazine and at least one film forming agent selected from the group consisting of citrate polyesters and polyamides with the proviso that the composition is free of octocrylene, ethylhexyl triazone, diethylamino hydroxybenzoyl hexyl benzoate and ethylhexyl methoxycinnamate and wherein the sunscreen composition further comprises one or more alkanediols.

2. The sunscreen composition according to claim 1, wherein the amount of bis-ethylhexyloxyphenol methoxyphenyl triazine is selected in the range from 0.1 to 10 wt.-%, preferably from 0.5 to 5 wt.-%, most preferably from 1 to 5 wt.-%, based on the total weight of the composition.

3. The sunscreen composition according to claim 1 or 2, wherein the amount of the at least one film forming agent selected in the range from 0.1 to 10 wt.-%, preferably from 1 to 5 wt.-%, most preferably from 2.5 to 5 wt.-%, based on the total weight of the composition.

4. The sunscreen composition according to any one of the preceding claims, wherein the citrate polyester is selected from the group consisting of octyldodecyl citrate crosspolymer, stearyl/octyldodecyl citrate crosspolymer and behenyl/octyldodecyl propanediol citrate crosspolymer as well as mixtures thereof, preferably the citrate polyester is stearyl/ octyldodecyl citrate crosspolymer.

5. The sunscreen composition according to any one of the preceding claims, wherein the polyamide is selected from the group consisting of polyamide-3, polyamide-4 and polyamide-8, as well as mixtures thereof, preferably the polyamide is polyamide-8.

6. The sunscreen composition according to any one of the preceding claims, wherein the one or more alkanediols are selected from the group consisting of 1,3-propandiol, butylene glycol and 1,2-butandiol, preferably from 1,3-propandiol and butylene glycol, most preferably butylene glycol is used as sole alkanediol.

7. . The sunscreen composition according to any one of the preceding claims, wherein the amount of alkanediol(s) is selected in the range from 1 to 10 wt.-%, preferably from 1.5 to 7.5 wt.-%, most preferably from 2 to 6 wt.-%, based on the total weight of the composition.

8. The sunscreen compositions according to any one of the preceding claims, wherein the composition is an O/W emulsion comprising an oily phase dispersed in an aqueous phase in the presence of an O/W emulsifier, preferably in the presence of a cetyl phosphate emulsifier, most preferably in the presence of potassium cetyl phosphate.

9. The sunscreen composition according to any one of the preceding claims, wherein the composition further comprises one or more of dicaprylate/dicaprate, phenethyl benzoate, C₁₂-C₁₅ alkyl benzoate, dibutyl adipate, diisopropyl sebacates, dicaprylyl carbonate, di-C₁₂-₁₃ alkyl tartrates, hydrogenated castor oil dimerates, triheptanoin, C₁₂-₁₃ alkyl lactates, C₁₆₋₁₇ alkyl benzoates, propylheptyl caprylates, caprylic/capric triglycerides, diethylhexyl 2,6-naphthalate, octyldodecanol, ethylhexyl cocoatesdibutyl adipate, preferably of dicaprylyl carbonate, phenethyl benzoate, C₁₂-C₁₅ alkylbenzoate, caprylyl carbonate, capric/caprylic triglyceride, most preferably of dicaprylyl carbonate, dibutyladipate and C₁₂-C₁₅ alkylbenzoate.

10. The sunscreen composition according to any one of the preceding claims, wherein the composition further comprises one or more of ethanol, phenoxyethanol and ethylhexylglycerin, preferably phenoxyethanol and ethylhexylglycerin.

11. The sunscreen composition according to any one of the preceding claims, wherein the composition further comprises one or more of xanthan gum, crosslinked acrylate/C₁₀-C₃₀ alkyl acrylate polymer, hydroxyethyl acrylate/sodium acryloyldimethyl taurate copolymer, preferably xanthan gum and hydroxyethyl acrylate/sodium acryloyldimethyl taurate copolymer.

12. The sunscreen composition according to any one of the preceding claims, wherein the composition further comprises one or more of behenyl alcohol, cetyl alcohol, cetearyl alcohol, stearyl alcohol and glyceryl stearate, preferably of behenyl alcohol, stearyl alcohol and cetearyl alcohol, most preferably stearyl alcohol.

13. A method of reducing the soaping effect of a sunscreen composition comprising bis-ethylhexyloxyphenol methoxyphenyl triazine, said method comprising the step of adding at least one film forming agent selected from the group consisting of stearyl/ octyldodecyl citrate crosspolymer and polyamide-8 into said compositions, preferably polyamide-8.

14. Use of at least one film forming agent selected from the group consisting of stearyl/ octyldodecyl citrate crosspolymer and polyamide-8 to reduce the soaping effect of bis-ethylhexyloxyphenol methoxyphenyl triazine in a cosmetic composition, preferably of polyamide-8.

15. The method according to claim 13 or the use according to claim 14, wherein the composition further comprises at least one alkandiol, preferably 1,3-propanediol and/ or butyleneglycol, most preferably butylene glycol is used as sole alkanediol in the composition.
